(19) **Europäisches Patentamt / European Patent Office / Office européen des brevets**

(11) **EP 3 224 801 B1**

(12) **EUROPEAN PATENT SPECIFICATION**

(45) Date of publication and mention of the grant of the patent:
**15.12.2021 Bulletin 2021/50**

(21) Application number: **15896597.0**

(22) Date of filing: **07.08.2015**

(51) Int Cl.:
**A61B 6/03** *(2006.01)* **G06T 5/00** *(2006.01)*

(86) International application number:
**PCT/CN2015/086397**

(87) International publication number:
**WO 2017/024451 (16.02.2017 Gazette 2017/07)**

(54) **MULTI-MODALITY IMAGING SYSTEM AND METHOD**

SYSTEM UND VERFAHREN ZUR MULTIMODALEN BILDGEBUNG

SYSTÈME ET PROCÉDÉ D'IMAGERIE À MODALITÉS MULTIPLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.10.2017 Bulletin 2017/40**

(73) Proprietor: **Shanghai United Imaging Healthcare Co., Ltd.**
**Shanghai 201815 (CN)**

(72) Inventors:
• **LV, Yang**
  **Shanghai 201815 (CN)**

• **LI, Hongdi**
  **Houston, Texas 77054 (US)**

(74) Representative: **Barker Brettell LLP**
**100 Hagley Road**
**Edgbaston**
**Birmingham B16 8QQ (GB)**

(56) References cited:
| CN-A- 103 079 470 | CN-A- 103 239 254 |
| US-A1- 2013 105 699 | US-A1- 2014 148 684 |
| US-A1- 2015 199 121 | US-A1- 2015 216 486 |

• **None**

Description

<u>TECHNICAL FIELD</u>

Embodiments of the present invention generally relate to a system and method for image generation in a combined examination using multiple imaging modalities.

<u>BACKGROUND</u>

Hybrid imaging devices, such as a combined Positron Emission Tomography (PET)-Magnetic Resonance (MR) system or a combined PET-Computed Tomography (CT) system, may be used to record both MR or CT measurement data and positron emission tomography measurement data simultaneously or asynchronously. The first imaging modality, conventionally either MR or CT, is able to depict the examination volume to be displayed in a first image, which may show the anatomical characteristics in a patient being examined. The second imaging modality, which may be, for example, PET, may be used to generate a further image. The further image may be used to show the distribution of a radioactive substance in the body and hence may depict biochemical and physiological characteristics of the patient. The MR or CT measurement data may be recorded either before, or after, or simultaneously with the recording of the PET measurement data.

The parallel creation of medicine images with two or more different modalities enables an improved evaluation of the state of the patient to be examined. For example, the PET images may be combined with or superimposed on the MR images to give a graphical illustration of various characters of the region of interest of the patient. Nevertheless, positioning of the patient may be an issue when performing hybrid medical imaging in a medical examination. During an examination, an object to be examined or a patient may need to be moved to different bed positions to perform different types of imaging of a same region of interest. With the conventional PET-MR imaging, a recording schedule, sometimes referred to as a "step and shoot" schedule, may be utilized to record PET measurement data successively at different bed positions. The recording time may be predetermined for each bed position.

The time for recording the PET measurement data in the conventional PET-MR hybrid system tends to be relatively long, lasting approximately 5 to 15 minutes per bed position. It might cause some issues to a patient lying on the bed, for example, to one with claustrophobia. One proposed solution is a more targeted and accurate examination procedure, by using the so-called "scout image." For instance, a scout image may be used to locate a potential malign area of the patient. The shooting of a scout image tends to be rather quick, thereby reducing the dose and exposure the patient is subjected to, and allowing the doctor to examine the target more closely.

Multi-bed MR or CT images are sometimes used as a scout image. See, for example, U.S. Patent Application Publication No. 2007/0173716, describing that the scout images shot at various bed positions are combined together to generate a whole body image of a patient. It may be used in later procedure of medical examination. Nevertheless, there is a need for a faster and more convenient generation of a scout image, using different modalities, such as PET.

The document US 2013/105699 A1 discloses the setting of image conditions of a PET scout scan.

<u>SUMMARY</u>

Embodiments of the present invention generally relate to a system and method for carrying out a combined examination using two imaging modalities. At least one embodiment of the invention generally relates to a method for producing and processing scout imaging data with a hybrid imaging device, which is able to perform and prepare emission tomography as a first imaging modality. At least one embodiment of the invention also generally relates to a hybrid imaging device. Hybrid imaging devices of this kind are, for example as combined PET-MR system or combined PET-CT system or combined SPECT-MR system or combined SPECT-CT system.

It is an object of the present disclosure to provide a scout image processing system, and scout image processing methods. The scout image processing methods may be applicable for nuclear medicine imaging system. The nuclear medicine imagining system may further be a hybrid imaging system. For example, a PET-MR system, or a PET-CT system, or a SPECT-MR system, or a SPECT-CT system. The scout image may be a PET image. The generation of the PET scout image may be within five to fifteen seconds. Optionally, the generation of the PET scout image may be based on the usage of landmarking or indicators.

The hybrid system disclosed herein provides a method and means for modeling the point spread function (PSF) such that the point spread function may be used to provide improved image reconstruction in an acceptable processing time. The PET system models the PSF in conjunction with PET Time-of-Flight (TOF) data in list mode.

The hybrid system disclosed herein provides a method and means for image reconstruction utilizing estimate of point spread function in certain iterative type algorithms. The iterative algorithm may be a maximization likelihood expectation maximization (MLEM) algorithm. The iterative algorithm may be a ordered subsets expectation maximization (OSEM) algorithm.

The PET system disclosed herein provides procedure of medical examination generating PET scout imaging according to one embodiment of the present invention. The procedure of the medical examination may consist of steps of data acquisition, histogramming, and transmission/attenuation, normalization, attenuation correction, renormalization, scatter correction, image reconstruction, and image display.

## BRIEF DESCRIPTION OF THE DRAWINGS

The accompanying drawings, which are incorporated in and constitute a part of this specification, illustrate several aspects described below.

FIG.1 is a diagram illustrating a PET/MR imaging system according to an embodiment of the present invention;
FIGs.2-A and 2-B illustrate multiple detector rings in exemplary 3-D PET systems;
FIG.3-A illustrates noncolinearity of 511 keV annihilation photons;
FIG.3-B illustrates an exemplary line-of-response in a PET system;
FIG.4 illustrates the principle of a rebinning algorithm;
FIG.5 is a representation of a PET scout image data recorded at different bed positions;
FIGS.6-A to 6-D illustrate representative lines of response for a four-ring scanner;
FIG.7 is a flowchart illustrating a process for obtaining a PET scout image according to an embodiment of the present invention;
FIG.8 is a schematic representation of a medical examination process utilizing PET scout imaging according to one embodiment of the present invention;
FIG.9 is a diagram illustrating a configuration of an apparatus for generating a combined or superimposed image according to an embodiment of the present invention;
FIG.10 is a flowchart illustrating a process of generating an image utilizing point spreading function (PSF) method according to an embodiment of the present invention;
FIG.11 is a flowchart illustrating an exemplary process for reconstructing PET scan images according to an embodiment of the present invention;
FIG.12 is a flowchart of the process for acquiring nuclear medicine image data according to an embodiment of the present invention;
FIG.13 is a block diagram of the parallel/pipelined architecture for PET image reconstruction.
FIG.14 illustrates the spatial invariance of two-dimensional and three-dimensional PET imaging with X-ray transforms.

## DETAILED DESCRIPTION

After reading this description, it will become apparent to one skilled in the art how to implement the disclosure in various alternative embodiments and alternative applications. However, not all embodiments of the present disclosure are specifically described herein. It will be understood that the embodiments are presented by way of example only, and not limitation. As such, this detailed description of various alternative embodiments should not be construed to limit the scope or breadth of the present invention as set forth below.

It is to be understood that the aspects described below are not limited to specific systems, methods of making such systems or uses thereof as such may of course vary. It is also to be understood that the terminology used herein is for the purpose of describing particular aspects only and is not intended to be limiting.

According to the specifications in the present application, unless otherwise specified in the content, articles such as "a", "an", and/or "the" do not necessarily indicate single forms, and also include plural forms. Generally, expressions such as "include" or "comprise" are only used to indicate numbered steps or elements. However, listing of these steps and elements is not exclusive, and methods or devices may include other steps or elements.

It is to be understood that the term PET refers to Positron Emission Tomography, and the term CT refers to Computed Tomography, and the term MR refers to Magnetic Resonance, and the term SPECT refers to Single-photon Emission Computed Tomography.

So-called "hybrid modalities," such as, for example, PET-CT, SPECT-CT, PET-MR and SPECT-MR, have attracted a lot of attention in the field of medical imaging in recent years. The advantages of such combinations include the connection of one modality with high local resolution (e.g., MR or CT) to a modality with high sensitivity (e.g., SPECT or PET). Reference is made below to a combined PET-MR or PET-CT system for illustration purposes. Embodiments of the present invention may however generally be used for all forms of hybrid modalities or associated measuring methods. For example, embodiments of the present invention may be used in a SPECT-MR hybrid system, where an appropriate accommodation or change may be made within the present invention as set forth below.

Hybrid imaging devices, such as a combined PET-MR system or PET-CT system, may be used to record both MR or CT measurement data and positron emission tomography measurement data simultaneously or asynchronously. In some embodiments, the first imaging modality, either MR or CT, may depict the examination volume to be displayed in a first image, which may show the anatomical characteristics in an object to be examined. The second imaging modality, e.g., PET, may be used to generate a further image. The further image may be used to show the distribution of a radioactive substance in the body of the object and hence may depict biochemical and physiological characteristics of the object. The MR or CT measurement data may be recorded either before, or after, or simultaneously with the recording of the PET measurement data.

In other embodiments, the first imaging modality may be PET, showing the distribution of a radioactive substance in the body of the object and is may depict biochemical and physiological functions (functional imaging) of the object. The second imaging modality may be used to generate a further image. The second imaging modality may be a MR or CT imaging device, configured to show the anatomical relationships in the object being examined.

An MR system may include a control unit, a measuring unit, and an imaging unit. In some embodiments, a measuring program may be run on the control unit, causing the measuring unit to be activated and record MR signals according to the planned measuring sequence. In some other embodiments, a measuring program may include a number of program steps and optionally measuring breaks, during which an operator may, for example, adjust the position of a bed where a patient is located or lies, or the height of a patient support, or administer contrast agent to a patient. In some embodiments, each program step may be assigned a measuring protocol, which controls the physical parameters of the measuring unit for measuring purposes.

PET may use the characteristics of the positron emitter and positron annihilation to assess the functioning of organs or cell regions quantitatively. For this purpose, corresponding radiopharmaceuticals may be administered to the patient before the medical examination. During the decay process of radiopharmaceuticals, the radionuclides emit positrons, the positron, after travelling a short distance, may interact with an electron, the process is so-called annihilation. By detecting these annihilation "event pairs" for a period of time, the isotope distribution of radiophamaceuticals in a cross section of the body of a patient may be reconstructed. These events may in turn be mapped within the patient's body, thus allowing for the quantitative measurement of metabolic, biochemical, and/or functional activity in volumes of interest in vivo.

In some embodiments, PET images (often in conjunction with an assumed physiologic model) may be used to evaluate a variety of physiologic parameters, including but not limited to glucose metabolic rate, cerebral blood flow, tissue viability, oxygen metabolism, and in vivo brain neuron activity.

In some embodiments, the annihilation "event pairs" may be captured utilizing two opposing PET detector blocks within a specific time window (coincidence measurement), wherein the annihilation location may be determined at a position on the connecting line between two detector blocks. In other embodiments, a PET detector may have multiple pairs of detection blocks, each pair of detection block flanking the sample area with their scintillator faces opposing one another. The plurality detection blocks of a PET detector may form various different configurations, including but not limited to the cubic column configuration and the barrel-shaped configuration. For instance, in the cubic column configuration, four detection blocks form two opposing pairs, each pair flank the sample area thus forming the cubic column; an angle between adjacent detection blocks is 90 degree. Whereas in the barrel-shaped configuration, eight detection blocks form four opposing pairs, each pair flanks the sample area thus forming the barrel; the eight detection blocks distribute even across a 360-degree circle. Other possible configurations of a PET detector may be used in connection with the present system.

In some embodiments, so-called tracers may be used to display different biological characteristics in the PET data, thereby further optimizing the region of interest in one imaging process, which are then analyzed in subsequent examinations.

In some embodiments, the PET image may be used as a scout image, or a pre-scan image, to illustrate the volume of interest, or region of interest. The PET scout image may be used to guide a doctor to proceed in further medical examination, for example, a hybrid PET/MR examination. The PET scout image may be produced in a short time window. For example, the time window may be a couple of seconds to tens of minutes. The time window may be tens of seconds to a couple of minutes. The time window may be 5 seconds to 15 seconds.

In some other embodiments, the PET scout image may be reproduced using various image reconstruction algorithms. For example, the PET scout image may be reproduced using OSEM (Ordered Subset Expectation Maximization) algorithm, or MLEM (Maximum Likelihood Expectation Maximization) algorithm, or 3DRP algorithm.

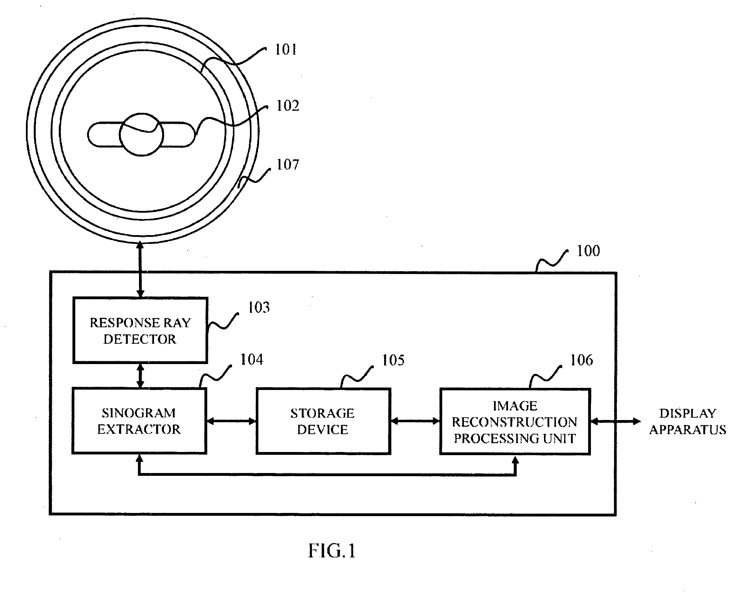

FIG.1 is a diagram illustrating a hybrid PET-MR imaging system according to some embodiments of the present invention.

The apparatus 100 for providing PET imaging may detect response rays in response to gamma photons that may be irradiated from a measurement object 102 through a photography apparatus 101. An MR imaging device 107 may be located outside the photography apparatus 101. The apparatus 100 may generate sinograms, and may obtain a PET scout image using the generated sinograms. The PET scout image obtained may be output into a display apparatus.

The apparatus 100 for providing PET imaging may include a response ray detector 103, a sinogram extractor 104, a storage device 105, and an image reconstruction unit 106.

The response ray detector 103 may detect response rays in response to gamma photons irradiated from a measurement target. In some embodiments, the measurement target may be an object. In other embodiments, the measurement target may be a patient. The sinogram extractor 104 may extract sinograms from the detected response rays. In some embodiments, a plurality of response rays may be converted into sinograms. Various ways of converting response rays into sonograms are known to those of ordinary skill in the art, including but not limited to the list-mode way.

The storage device 105 may store the extracted sinograms from the sinogram extractor 104. In some embodiments, the storage device 105 may perform some preliminary operations on the extracted sinograms obtained from the sinogram extractor 104. Merely by way of example, the storage device 105 may convert the extracted sinograms into high resolution sonograms, using a maximum a priori expectation maximization algorithm (MAP-EM) such as a maximum likelihood expectation maximization (MLEM) algorithm or an ordered subset expectation maximization (OSEM) algorithm. The storage device 105 according to some embodiments of the present invention may store a set of high resolution sinograms.

According to some embodiments of the present invention, a non-negative characteristic may be maintained in the computation of sinograms, by applying the OSEM algorithm that may be based on the MLEM algorithm. Further, the non-negative characteristic of a sinogram may be maintained by applying the MLEM algorithm or OSEM algorithm to the storage device 105, or the image reconstruction unit 106.

The image reconstruction processing unit 106 may reconstruct a PET scout image from the stored sonograms in storage device 105. In some embodiments, the image reconstruction processing unit 106 may reconstruct a PET image using both sinograms generated in sinogram extractor 104 and/or a set of the stored sonograms in storage device 105.

In some embodiments, the image reconstruction unit 106 may reconstruct the PET image, using an analytic reconstruction algorithm, or an iterative reconstruction algorithm. In some embodiments, the image reconstruction unit 106 may reconstruct the PET image using the analytic reconstruction algorithm similar to a filtered-back projection (FBP) algorithm. In other embodiments, the image reconstruction unit 106 may reconstruct the PET image using the analytic reconstruction algorithm similar to a three dimensional re-projection (3DRP) algorithm. In some embodiments, the image reconstruction unit 106 may reconstruct the PET image using the iterative reconstruction algorithm similar to an ordered-subset expectation maximization (OSEM) algorithm. In other embodiments, the image reconstruction unit 106 may reconstruct the PET image using the iterative reconstruction algorithm similar to a maximum likelihood expectation maximization (MLEM) algorithm.

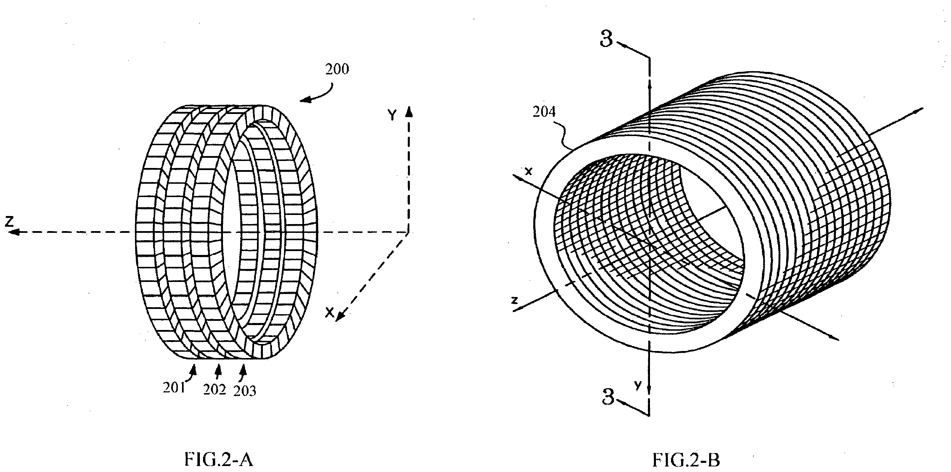

Apart from the concise presentation of system and procedure involved in producing PET image above, a more systematic and detailed explanation of the imaging process and apparatus involved in obtaining a PET image is presented subsequently. In some embodiments, the photograph apparatus 101 in Fig 1 may be a PET scanner. An exemplary PET scanner with multiple detector rings is illustrated in FIG. 2-A, where the PMTs are not shown. As illustrated, the PET scanner 200 may include three detector rings 201, 202 and 203. FIG. 2-B is a perspective view of the detector rings of an PET scanner according to some embodiments. FIG. 2-B illustrates a scanner 204 with 16 detector rings. If the detectors that have detect a pair of gamma rays are located on the same ring, the coincidence plane, which is a trans-axial plane, may be referred to as a direct plane. If the detectors that have detect a pair of gamma rays are located on different rings, the coincidence plane, which is an oblique plane, may be referred to as a cross plane.

The detector rings may capture the gamma quanta ("photon") produced in the annihilation, caused by the interaction between a positrons and electron. Since there may be some residual momentum associated with the positron, the two annihilation photons may emit at an angle slightly deviated from 180°. In some embodiments, a straight line connecting two pairing detectors that have detected these photons may be slightly deviated from the original annihilation line of the emitted photons. In some embodiments, the deviation may be $\pm 0.25°$ or lower.

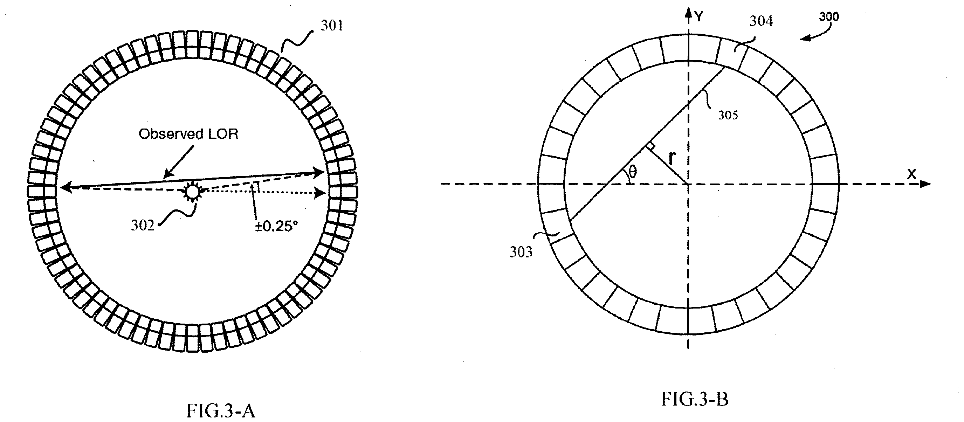

As a result, the observed line of response (LOR) between the two detectors may not intersect the point of annihilation, but may be somewhat displaced from it, as illustrated in Fig. 3-A. This error (Re) may reduce the spatial resolution of the scanner and deteriorate with the increase in the distance between the two pairing detectors. This error may be calculated by using the point spread function (PSF) method. In some embodiments, if D is the distance (e.g., in centimeter) between the two detectors (i.e., detector ring diameter), the error may be calculated from the point-spread-function (PSF) as follows: Re = 0.0022D.

FIG. 3-B illustrates an exemplary line-of-response (LOR) in a PET system. In some embodiments, data associated with coincidence events may be stored in the form of sinograms based on their corresponding LORs. Merely by way of example, in a PET scanner like the one illustrated in FIG. 3-B, if a pair of coincidence events are detected by two opposite detectors 303 and 304, an LOR may be established as a straight line 305 linking the two detectors. Two coordinates (r, 0) may be used to identify this LOR, wherein r is the radial distance of the LOR from the center axis of the detector ring 300, and 0 is the trans-axial angle between the LOR and the X-axis. The detected coincidence events may be recorded as a 2-D matrix $\lambda(r, \theta)$. As the PET scanner continues to detect coincidence events along various LORs, these events may be binned and accumulated in their corresponding elements in the matrix λ(r, θ). The result may be a 2-D sinogram matrix λ(r, θ), each element of which holds an event count for a specific LOR. In a 3-D PET scanner, four coordinates (r, θ, φ, z) may be used to define an LOR, wherein the third coordinate φ is the axial angle between the LOR and the center axis of the detector rings, and z is the distance of the LOR from the center of detector along the Z-axis. In some embodiments, the third and fourth co-ordinates may be combined into one variable, v, which may define both φ and z coordinates. In this case, the detected coincidence events may be stored in a 3-D sinogram matrix λ(r, θ, v).

In addition to the true coincidence events described above, two other types of coincidence events may also be detected by the PET scanner. These extra events may complicate the data collection and image reconstruction process. The first type of confounding events arises because the annihilation photons may exhibit scattering effect as they emit out of the patient. In the case where one or both of the annihilation photons scatter, and are subsequently detected in coincidence, they may register a coincidence event along an LOR that does not correspond to the site of the annihilation event. These events may be referred to as scattered coincidences. In some cases, a scattered coincidence may differentiate from a true coincidence in that the scattered photons have energy less than 511 keV. However, in practice, the energy of each detected photon may fail to be measured exactly. As a result, some scattered photons together with some scattered coincidences, may be accepted by the scanner. The scattering effect may be considered in the image reconstruction process, by using the point spread function method, a renormalization method, or the like, or a combination thereof.

The second type of confounding coincidence event may arise from the substantially simultaneous detection of two photons that arose from two different annihilation events occurred simultaneously. These coincidences may be referred to as "random coincidences." The contribution of random coincidences may be reduced by reducing the timing window used to define the simultaneous detection of the coincident photons, but some random coincidences may still be accepted by the scanner. According to some embodiments of the present invention, the rate of random coincidence acceptance may be estimated by some methods, including but not limited to a delayed window method, the randoms from singles method, or the like, or a combination thereof.

In PET, as an exemplary embodiment of the present invention, the recording of sinogram data may be achieved as in the form of "list mode," or listed in a list-mode file according to the time or location of arrival (of the photons to the detectors), or the time or location of detection. The list-mode data format means the raw data, i.e., the events detected by the detectors and recorded together with a time stamp. In some embodiments, the measurement data is recorded in a pre-processed format, which may be convenient for emission tomography reconstruction. Merely by way of example, in a certain list mode data format, each of the events is identified by the imaging detector numbers, and x and y coordinates. In some examples, the data in the list mode data format may be given by (ia, ib, ra, rb), wherein (ia, ib) is the labelling of the pair of detector rings associated to a LOR arizumuthlly, and (ra, rb) is the labelling of the pair of detector rings above longitudinally.

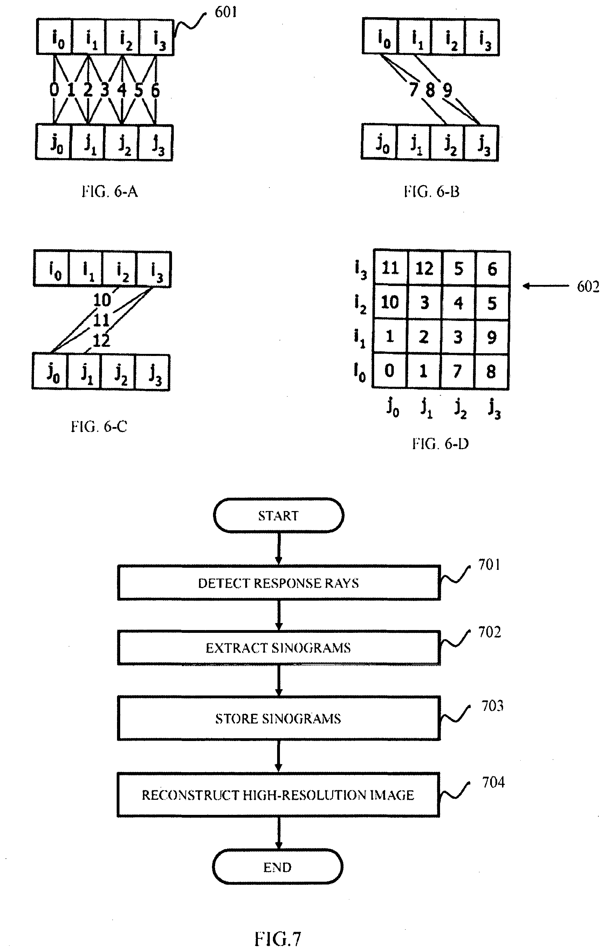

In some embodiments of the present invention, FIGS. 6-A through 6-C illustrate the list mode data format for a four-ring scanner 601. FIG. 6-A shows the direct coincidence planes 0, 2, 4, 6 and the pair of oblique coincidence planes 1, 3, 5. FIG. 6-B shows the oblique coincidence planes 7, 8, 9. FIG. 6-C shows the corresponding oblique coincidence planes 10, 11, 12. FIG. 6-D illustrates the placement of those coincidence planes on a matrix 602, which is a graphical representation of the planes of response which get grouped together to reduce the data set size in 3D PET. Referring to the matrix 602 of FIG. 6-D, the coincidence planes 0 through 12 are indicated by the numbers in the cell corresponding to the coordinates of the rings i, j illustrated in FIGS. 6-A through 6-C.

As may be seen in FIGS. 6-B and 6-C, coincidence planes 8 and 11 define the maximum angle from the direct planes that an oblique plane will have for the number of rings in the scanner 601. This angle may be referred to as the acceptance angle.

In some embodiments, after the acquisition of list-mode data, a streamlined procedure for processing the acquired data, either synchronously or asynchronously, may be presented subsequently. Merely by way of example, the streamlined procedure may start by performing histogramming, normalization, transmission/attenuation, attenuation correction, scatter correction, rebinning, image reconstruction, and image display. In some other examples, the procedure of rebinning may be omitted, and image reconstruction may be performed directly after the completion of scatter correction. In some embodiments, normalization may be performed simultaneously with image reconstruction.

For the hybrid PET/MR system, attenuation correction may be needed. In some embodiments, although the MR coils may be partially radiation-transparent, the MR coils may degrade the image quality if the data is not corrected for attenuation caused by the MR coils, as well as by other objects in the vicinity including, e.g., the bed (where a patient being examined lies).

There may be several ways to perform attenuation correction. In some embodiments, PET/MR hybrid systems may use MR scans for attenuation correction of PET images. In some embodiments, for the hybrid PET/MR system, the patient attenuation map may be developed based on the PET scout images. The PET patient scout image may be segmented, the various tissues and organs identified, and appropriate attenuation coefficients applied.

Two types of image reconstruction may be available on PET scanners: analytic techniques and iterative techniques. Analytic techniques include but are not limited to the filtered back-projection (FBP) techniques. The FBP method may be used for transmission tomography systems such as computed tomography (CT), since its structure is simple and the computational time is relatively short. Iterative techniques may be used for a PET system. Exemplary iterative techniques include but are not limited to the maximum likelihood expectation-maximization (MLEM), which incorporates the Poisson nature of raw data into the algorithm.

As the expectation maximization (EM) algorithm iteratively guesses an image estimate, the low frequency components of the image appear within the first few iterations. As the maximum likelihood (ML) estimate proceeds, more and more high frequency definition is resolved in the image, effectively adding more variance to the reconstruction. This variance may be reduced by stopping the algorithm early or by post-smoothing the reconstruction. The convergence rate of MLEM may be image dependent. In some embodiments, MLEM may needs approximately 20-50 iterations to reach an acceptable solution.

In some embodiments, ordered subsets expectation maximization (OSEM) algorithm may be applied since it provides an order-of-magnitude acceleration over MLEM. Differently from MLEM using all of the projecting data to updating image data, the OSEM groups projection data into an ordered sequence of subsets (or blocks) and progressively processes every subset of projections in each iteration process. Every subset may update the whole image intensity data and the image intensity data is updated k times when all the projecting data are used (assume there are k subsets), and this is called a step. Image data is updated once in each iteration step of MLEM, but k times in OSEM.

An exemplary illustration of OSEM Reconstruction Algorithm is as follows:

$S_1$, $S_1$, ... ,$S_L$ is L sorted subsets, the disclosure adopt the orthogonal partition, so the number of projection data(detectors) in every subset is $J = \dfrac{M}{L}$ . $X^0$ denotes the initialized image used the FBP algorithm. $X^i$ denotes the image after iteration i. The concrete step of developed OSEM algorithm is:

1. Set i=0, initialize the image X = $X^0$ with FBP algorithm;
2. Iterate the following steps until image $X^i$ satisfies the convergence request:

a) Set X = $X^i$, i=i+1;
b) For each subset: $S_l$, $l$ = 1,2, ..., L, process the following iteration:

(1) Project: For every detector i (i=1,2,...,M) in subset $S_l$, assume $a_{ij}$ denotes the $x_j$'s contribution to detector i, calculate its mathematical expectation:

$$y_i^l = \sum_{j=1}^{N} a_{ij}\, x_j^l \qquad\qquad (1)$$

(2) Back Project: Update the image at iteration $l$:

$$x_j^{l+1} = \frac{x_j^l}{\sum_{j=1}^{N} a_{ij}} \times \sum_{j=1}^{N} \frac{y_i a_{ij}}{\sum_{j=1}^{N} a_{ij} x_j^l} \qquad j = 1,2, ... , N \qquad (2)$$

(3) $l = l + 1$;

The OSEM algorithm as described herein only uses part of projecting data, the subset $S_l$, at equation (1), but updates the whole image X in equation (2). (c) If $l = L + 1$, then $X^i = X^L$, reiterate by repeating step a) and b)).

Those skilled in the art will recognize that processing stages may be modified or additional processing stages (various corrections, such as arc correction, etc.) may be added to the algorithm without departing from the scope of the present invention. For example, in Step 1 above, the initialization of the image may be given by setting the initial image to have value one for each pixel of the image. As another example, the OSEM algorithm may be given by utilizing the following steps:

$$f_j^{(n+1)} = \frac{f_j^{(n)}}{\sum\limits_{i_k \in S_k} M_{i_k j} \left(\sum\limits_t W_t\right)} \sum\limits_{k \in S} M_{i_k j} \left(\sum\limits_t W_t \frac{m_{ik}}{\sum\limits_{j_p \in L_{i_k}} M_{i_k j_p} \left(\sum\limits_t W_t f_t^{(n)}\right)}\right)$$

Where $S_k$ is the k-th subset, $L_{i_k}$ is the $i_k$-th LOR in the k-th subset, $W_f$ is the corresponding point spread function (PSF),

$M_{ikj}$ is the value of the system matrix corresponding to the LOR $L_{i_k}$ and the j-th pixel, $f_j^{(n)}$ is the value of the j-th pixel after the n-th iteration, $m_{i_k}$ is the actual counting number on the $i_k$-th LOR after the procedure of normalization.

Apart from the iterative techniques that may be used for image reconstruction, the analytical techniques may also be used for image construction. Merely by way of example, three dimensional re-projection (3DRP) algorithm may also be used for obtaining PET scout image.

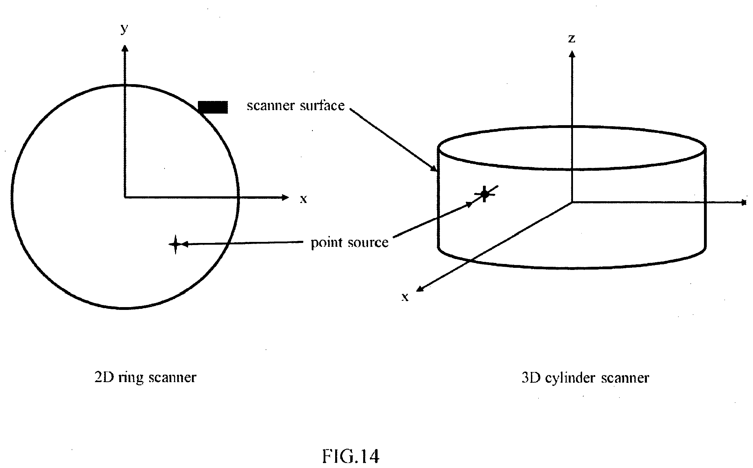

To explain the mechanism of 3DRP algorithm, one note that the spatially-varying scanner response can be understood as the consequence of the finite axial extent of the scanner, leading to truncated projections. In a two-dimensional ring scanner, the observed intensity of a point source will remain approximately constant, regardless of the position of the point source inside the scanner's FOV, as illustrated in FIG. 14. For a three-dimensional cylindrical scanner, however, with truncated projections the observed intensity of a point source will vary depending on the position of the point source inside the scanner's FOV, particularly as the point source moves axially (shown in FIG. 14).

Merely by example, the steps of the 3DRP method may be as follows:

(1) For the collected projection data, a three-dimensional low-statistics image is formed from the subset of projection data for which the angles are small enough (e.g., below 20%, or below 15%, or below 10%, or below 5%, or below 3%).
(2) This first image is then forward projected, or re-projected, onto the region of missing projection data in the remaining subset of the projection planes.
(3) The new set of projection data is filtered and back projected along with the original complete projection to form a high-statistics, three-dimensional image, using an appropriate filter (e.g., a high pass filter, a low pass filter, a Colsher filter, a Butterworth filter, a Hann filter).

In some embodiments, the measured data may be utilized to estimate a stacked (volumetric) set of two-dimensional transverse sinograms by using some form of signal averaging via three-dimensional X-ray transform. Such a procedure is called a rebinning algorithm. Each rebinned sinogram may be efficiently reconstructed with either analytic or iterative two-dimensional reconstruction methods. In addition rebinning may reduce the size of the data.

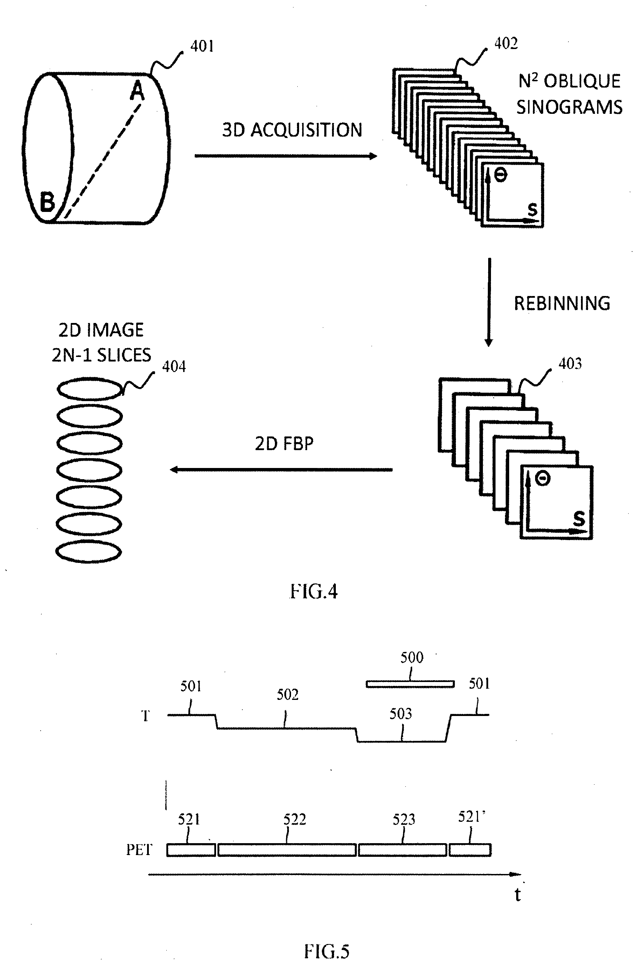

In some embodiments, the process of rebinning may be performed on the three-dimensional data by using two dimensional slicing technique. FIG. 4 illustrates the principle of a rebinning algorithm. Three-dimensional data may be acquired from the scanner and processed into $N^2$ oblique sinograms 402, where N represents the number of direct slices or sinograms for the scanned image. The oblique sinograms are rebinned into 2N—1 ordinary sinograms 403, which represent slices separated by one-half the axial distance between adjacent detector rings. The rebinned data 403 is converted to 2N—1 slices for the 3D image 404 by using a 2D numerical algorithm, including but not limited to the filtered back-projection (FBP) algorithm. The process of rebinning may be known to those with ordinary skill in the art.

In some embodiments, the single-slice rebinning (SSRB) algorithm may be used in the rebinning process, where the rebinned sinograms are formed from averaging all of the oblique sinograms that intersect the direct plane at the center of the transaxial field of view. In some embodiments, the Fourier rebinning (FORE) algorithm, which is based on a reasonably accurate equivalence between specific elements in the Fourier transformed oblique and transverse sonograms, may be used. Put in another way, the Fourier transformed oblique sinograms may be resorted into transverse sinograms and, after normalization for the sampling of the Fourier transform, inverse transformed to recover accurate direct sinograms.

Apart from the issue of image reconstruction for PET scout imaging, the issue of multiple scout images and their combination or fusion may also need to be addressed. Merely by way of example, during an examination, the object to be examined or the patient may need to be moved to different bed positions to ensure the imaging of region of interest. With the hybrid PET-MR imaging, a recording schedule, sometimes referred to as "step and shoot" schedule, may be utilized to record PET measurement data successively at different bed positions. The recording time may be predetermined for each bed position. In other embodiments, the recording time may be set by operators manually.

In some embodiments, CT pre-scan images or MR pre-scan images may be used as scout images. Scout images may be used to plan and monitor medical scan acquisition. Also, scout images may be used to shorten the time needed to obtain sufficient geometrical details, while keeping radiation dosage, if any, to acceptable levels. Merely by way of an example, in the case of using MR images as scout images, first a start position and an end position is set for obtaining a scout image based on a region of the object to be imaged. The scout image may be referred to as a scout localization image or localizer image. Scout images may be acquired before obtaining MR images for diagnostic purposes to determine an angle at which a cross-sectional image may be acquired. The scout image may also serve as an index for a position of MR image data. Scout images may be used to identify the location and overall shape of an internal organ or lesion and obtained at a lower resolution than a diagnostic MR image.

In some exemplary embodiment of the present invention, the scout image may be used to locate the volume of interest or region of interest of a patient. Merely by way of examples, in the case of using PET images as scout images, an exemplary volume of interest may be selected manually by the operator after reviewing the scout scan image. Optionally, the volume of interest may be selected automatically by the PET imaging system by comparing the scan data utilized to generate the scout image to historical scan data.

Further, the scout image may be used so that multiple scans of a patient may be performed with different sensitivities and resolutions.

By way of example, a scout image may be acquired in a configuration of higher sensitivity and lower resolution. In some embodiments, the first image may be of a kidney of a patient. Then, depending on the specific position of the patient, his size, his shape, and the distribution of gamma-ray attenuating tissues, the collimator configuration may be adjusted. The adjustment may improve the balance between sensitivity and resolution of the imaging being or to be taking. A second image may then be obtained during the same examination without removal of the patient. Based on the adjustment, this second image may be at a higher resolution but lower sensitivity than the first image.

Ever further, the scout image may be implemented by using landmarking to depict the relative locations among multiple scans of the patients. Merely by way of example, the method may include obtaining images to be used for landmark correction as described below. In some embodiments, calibration images are optionally obtained, which may be performed automatically or manually. For example, in PET scans with parallel imaging, a scout scan that spans a large region of a patient may be obtained as part of a "calibration" scan to acquire the calibration images.

The method may also include optionally acquiring localizer images, which may be performed automatically or manually. The localizer images may be used in localizing the region of interest of the patient. The localizer images may be acquired employing a hybrid modality imaging system. Further, the single modality imaging system may be a PET imaging system or an MR imaging system. The localizer images may be used to ensure that the region of interest, such as the cardiac region, for example, is located within the field of view of the one or more localizer images. The term field of view (FOV) may refer to physical dimensions of acquisition. For example, images or image volumes representative of a thoracic and/or cardiac region of the patient may be acquired such that the images include the heart. The localizer images may include scout images, locators, scanograms, plan scans, and the like.

In various embodiments, a 2D localizer image or a 3D localizer image may be acquired. The localizer images may be obtained in a sagittal plane, a coronal plane, an axial plane, a volume of interest, multiple volumes of interest, or in any plane or combination thereof.

It should be noted that if the localizer images are acquired using a multi-modality imaging system, a feature space in each of the acquired localizer images may be standardized in order to match data in the multi-modal space. The feature may be a characteristic point in the localizer image. The feature may also be a region in the localizer image with maximum gray level. Using the calibration and/or localizer images, one or more landmark positions in the images may be determined. Merely by way of example, based on a particular region of interest, such as the anatomy of interest, one or more landmark positions may be determined as described herein. In general, the localizer images may be processed, such as using suitable image segmentation or other decomposition methods to identify specific patient anatomy, which may then be used to identify one or more landmark positions. The identified one or more landmark positions may be one or more of a determined set of ideal landmark positions. Thus, in some embodiments, segmentation of the localizer images may identify sub-anatomies within the patient's body. Based on known relationships of the sub-anatomies (e.g., relative locations in the body) or statistics or other measurements, one or more landmarks may be determined. Accordingly, in some embodiments, correction may be provided by recognizing or identifying imaged landmarks and then choosing any ideal landmark, such as a midpoint between two identified landmarks (e.g., two organs).

In this way, an offset between the initial landmark and the identified landmark may be computed. Merely by way of example, a difference in the location of the landmark from the initial landmarking and the desired or ideal landmark determined from the calibration and/or localizer images is determined. For example, using the one or more calibration and/or localizer images, these images may be compared with images from the initial patient landmarking. The comparison may determine a difference between the calibration and/or localizer images and the initial landmark. For example, a pixel by pixel comparison of the images may be performed to determine an offset between the current and desired or ideal landmark(s).

In a related art PET apparatus, it may be difficult to take a scout image over a wide range. In order to set an FOV by using a scout image, the method described above may be repeated for N times on each image region.

A process of setting FOVs for an example including two image regions (N=2). An isocenter is set on an object with respect to the first image region, and then the isocenter is moved to an isocenter of a distinct part of PET imaging apparatus, e.g., a gantry. A start position and an end position for obtaining a scout image are set, and the scout image is acquired. A detailed FOV is set by using information obtained from the scout image, and examination is performed on the first image region. After completing the examination of the first image region, the same operations may be repeated for the second image region. The two scout images may then be combined together to form a scout image of the region of interest, offering more detailed and complete information on the particular body area of the patient.

In a hybrid medical imaging system, the different requirements of the modalities in respect to measurement planning may increase the difficulty in the planning of an optimal measuring sequence. For an optimal diagnostic evaluation of data records acquired using hybrid modalities, it is needed to prepare for and carry out the examination in an appropriate manner.

In some embodiments, sequential recording are used by both modalities. Merely by way of example, MR measurements and PET measurements may be planned one after the other. For example, MR examinations may deploy techniques, with which a fairly large region of the body may be examined, in that the patient bed with the patient supported thereon is passed through the magnet, with examinations being carried out in different bed positions. This makes it possible to examine various regions of the body which may be larger than the examination volume available in the system.

In some embodiments, a body region that is larger than the available image field, may be examined wherein a number of so-called levels are measured. The body region is divided into individual segments for measurement. In some embodiments, the measurement region may contain a number of sub-measurements in an associated bed position. By examining different body regions at different levels (bed positions or imaging regions), it is possible to record the body as a whole. The images recorded at each level may be associated with a set of measuring parameters including, for example, the echo time, the repetition time, the layer thickness, the number of layers, the voxel size, the layer orientation, etc.

Due to the different lengths of the dwelling times at different bed positions, at some bed positions, a smaller volume of data may be recorded, or the date recording may last for a shorter time, than at other bed positions. In some embodiments, it may be determined in advance a minimum quality in a reconstructed PET image to be achieved at each bed position. The minimum quality may be quantified by using some indicators or indices. For example, the contrast of the PET scout image may be used as indicator or index. In some other embodiments, the gray level of the PET scout image may be used as indicator or index for quantifying the minimum quality of the reconstructed PET image.

FIG. 5 shows an exemplary examination process, plotted against the time t, according to one embodiment of the present invention.

The upper part T of the image shows three different bed positions 501, 502, and 503, which are approached for the performance of a PET examination. These bed positions may, for example, correspond to an examination of the brain, the abdomen, and the legs of a patient.

The lower part PET of the image shows the recording of the PET measurement data 521, 521', 522, and 523. Hereby, the recording may take place in the background in what is known as the list-mode data format. The bed positions 501, 502, and 503 are recorded simultaneously so that, during the course of the further processing, it is possible to assign the PET measurement data 521, 521', 522, and 523 to the bed positions 501, 502, and 503 at which they are respectively recorded.

The recording of the PET measurement data 521, 521', 522, and 523 may be performed at the bed positions 501, 502, and 503. It is during the movement of the patient bed from one position to another that the recording of the PET measurement data 521, 521', 522, and 523 is interrupted. If there is a longer waiting time, the bed may also be moved specifically for the recording of PET measurement data to a position at which it is still needed to record or to supplement PET measurement data (not shown here).

Due to the different lengths of the dwelling times at various bed positions, at some bed positions, a smaller volume of data may be recorded, or the data recording may last for a shorter time, than at other bed positions.

However, it may be determined in advance a minimum quality of a reconstructed PET image to be achieved at each bed position, and the needed data volume or the needed recording duration (symbolized by the $\overline{500}$).

The example shows that, at the first bed position 501, the first PET measurement data 521 are recorded, the volume of which is lower than the needed data volume or acquisition time ($\overline{500}$).

At the second bed position 502, on the other hand, a sufficient volume of the second PET measurement data 522 are recorded.

At the third bed position 503, once again an insufficient volume of the third PET measurement data 523 are recorded. Therefore, immediately after the completion of the recording of the second PET measurement data 522, the patient remains at the third bed position 503 and the recording of the third PET measurement data 523 is completed until the needed data volume $\overline{500}$ of PET measurement data for the third bed position 503 has been achieved.

Then, the bed is moved back to the first bed position 501 in order also to record additional first PET measurement data 521', until the needed volume of PET measurement data $\overline{500}$ for the first bed position 501 has been recorded.

FIG. 7 is a flowchart illustrating a method of obtaining a PET scout image according to an embodiment of the present invention.

As illustrated, multiple sets of sinograms may be obtained using a photography apparatus by moving an entire detector or a bed, and a PET image may be reconstructed by applying an image reconstruction algorithm, such as OSEM algorithm, to the obtained sinograms.

In step 701, response rays may be detected in response to gamma photons that may be emitted from a measurement target, e.g., an object or a patient, or a portion thereof.

In step 702, sinograms may be extracted from the detected response rays.

In step 702, a set of sinograms may be obtained by moving the entire detector or the bed. In this instance, the set of the extracted sinograms may correspond to data measured directly in a PET system.

In step 703, the extracted sonograms may be stored. Optionally, in this step the extracted sinograms may be converted into high resolution sinograms.

In operation 704, a PET image may be reconstructed from the stored sinograms, or from the converted high resolution sinograms.

In operation 704, the set of the converted high resolution sinograms may be reconstructed to provide a PET scout image. The reconstruction may be achieved using an analytic reconstruction algorithm, or an iterative reconstruction algorithm.

In the PET system that may use a motion of the entire detector or a motion of the bed, an image having high resolution may be reconstructed, and a high resolution image may be obtained by applying an OSEM algorithm to the sinograms.

Also, according to some embodiments of the present invention, a non-negative characteristic of a PET sinogram may be maintained using a positive number only, in computation of sinograms, by applying the reconstruction algorithm that may be based on, e.g., the MLEM algorithm, OSEM algorithm, or the like.

In some embodiments, the apparatus for improving resolution may use a discrete wobble to decrease the blurriness of sinograms. Also, the image reconstruction process may involve at least one of the following features: automatically estimating a blur kernel of the PET image based on information measured in the PET detector; estimating a correlation between high resolution sinograms and normal sinograms based on at least one of the sinograms that may be measured in at least one wobble position; estimating a noise component that may enable the normal sinograms to be a random vector having a Poisson distribution and the high resolution sinograms.

In some embodiments, the image reconstruction process may include calculating at least one of the following matrices including, e.g., a motion matrix in at least one wobble position, a matrix indicating down-sampling, and a matrix indicating the difference in blurriness between the high resolution sinograms and the normal sinograms (based on the correlation between the estimated high resolution sinograms and the normal sonograms).

In some embodiments, the image reconstruction process may include selecting data in a bed position corresponding to at least one angle, using a Monte Carlo simulation, and may estimate a blur kernel based on the selected data.

In some embodiments, the image reconstruction process may include first calculating a part of a matrix indicating the blurriness and down-sampling with respect to at least one angle, among the sinograms, and may derive a remaining part of the matrix using the calculated result.

Further, the image reconstruction process may include calculating a unique solution according to regularization using, e.g., an MAP-EM algorithm with respect to a Poisson distribution, or a total-variation regularization algorithm.

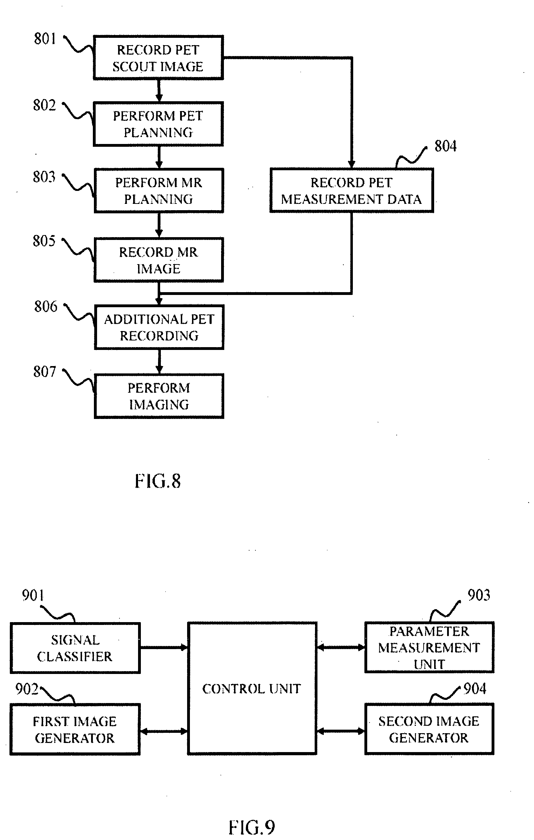

FIG.8 is a schematic representation of a process for medical examination of a patient utilizing PET scout imaging according to one embodiment of the present invention.

Firstly, a PET overview image (so-called scout image) of the patient is recorded (step 801). This PET overview image may be recorded with little time expenditure, for example within 5 seconds per bed position. PET measurement data may be recorded in the background during this recording (step 804). It may be used to plan for the subsequent acquisition.

Optionally, the PET overview image may also be used to perform an attenuation correction during the reconstruction of the PET image or to determine the minimum volume of PET measurement data to be recorded per FOV.

Subsequently, the FOVs for the PET measurement may be determined based on the PET overview image (step 802). PET measurement data which were already recorded during the PET overview image may be assigned to corresponding FOVs.

In a further step 803, the patient and disease-specific planning of the MR measurement may be performed with reference to the PET overview image. In this planning phase, which takes a certain amount of time, recording of PET measurement data may take place (step 804), the measurement data may be recorded in the background.

If desired, in this phase the patient or the patient bed may have already be moved to a different position, e.g., where PET measurement data are still missing.

This is followed by the recording of conventional PET/MR measurement data (step 805). During this recording of MR measurement data, PET measurement data continue to be recorded in parallel.

When the recording of the MR measurement data is complete, optionally further PET measurement data are recorded (step 806) until sufficient PET measurement data are recorded for all FOVs, such as were defined in step 802.

After the recording of measurement data are completed, there may be a further harmonization of the PET measurement data. Individual MR or PET images and/or a hybrid image may be reconstructed from the MR measurement data or from the PET measurement data (step 807).

The exemplary embodiments should not be understood as a restriction of the invention.

FIG.9 is a diagram illustrating a configuration of an apparatus for generating a combined or superimposed image according to an embodiment of the present invention.

The apparatus may detect response rays in response to radioactive rays that may be irradiated to a measurement target, may extract sinograms from the detected response rays, and may reconstruct a PET scout image by converting the extracted sinograms into high resolution sinograms.

As illustrated in FIG. 9, the apparatus for generating the image may include a signal classifier 901, a first image generator 902, a parameter measurement unit 903, and a second image generator 904.

The apparatus may apply input signals through a motion of an entire PET detector or a bed motion, may generate a first image set based on the input signals that may be classified based on a position of the PET detector, may measure a point spread function (PSF) based on the first image set, and then may generate second image information that may be improved through, e.g., an OSEM imaging technique.

The second image generator 904 of the apparatus for generating the image may generate the second image information that may have better resolution, using the PSF as a blur model.

The apparatus for generating the image may generate the second image information corresponding to the high resolution image information, by applying, e.g., the following OSEM algorithm.

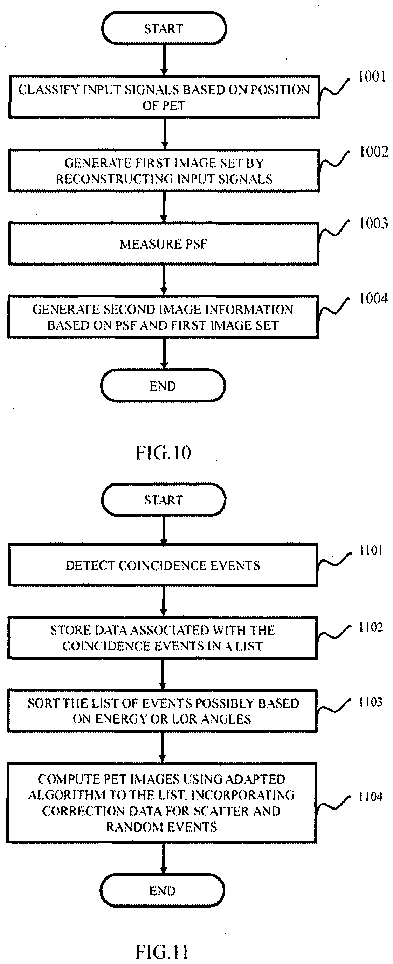

A method that may generate an improved image through the OSEM imaging technique, using the apparatus for generating the image according to this embodiment will be further described with reference to FIG. 10.

FIG. 10 is a flowchart illustrating a process for generating an image according to an embodiment of the present invention.

The apparatus for generating the image may generate image information based on, e.g., an OSEM algorithm, using input signals that may be applied when a measurement target (e.g., an object or a patient, or a portion thereof) passes through a PET detector.

An apparatus as disclosed herein may generate the image information by applying the input signals that may be measured through a circular movement, such as a wobble motion of an entire PET detector or a bed where a patient may lie.

Input signals acquired through the motion of the entire PET detector or the motion of the bed may be classified (e.g., using the signal classifier 901), based on the positions of the PET, in order to generate image information by applying an OSEM algorithm, in step 1001.

Then, A first image set may be generated by reconstructing the classified input signals (e.g., using the first image generator 902), in step 1002.

In this instance, the first image set may correspond to a set of PET images. Second image information may be generated based on the first image information.

For example, the second image information, the information of a sheet of a $128 \times 128$ image, may be generated using the first image set including information of four sheets of $64 \times 64$ images.

In operation 1003, the apparatus for generating the image according to the embodiment may measure a point spread function (PSF) based on the statistics of previous measurement of PSF, using the parameter measurement unit 903.

In operation 1004, the second image generator 904 of the apparatus for generating the image may generate second image information by applying the OSEM algorithm based on the PSF and the first image set. Here, the second image information according to the embodiment described with reference to FIG. 9 may correspond to information with respect to a high resolution image.

FIG. 11 is another flowchart illustrating an exemplary process for reconstructing PET scout images according to an embodiment of the present invention.

In step 1101, coincidence events may be detected in a PET scanner. In some embodiments, individual detectors in the PET scanner may detect gamma photons (individual events) resulting from positron annihilations in the imaged object. These individual events may be recorded if they fall within certain energy window(s). In some data formats, for example the list mode, each event may be assigned a location ID and a time-stamp to indicate the detector in which it was detected and the time of detection, respectively. Then the event data may be processed to identify coincidence events. In some embodiments, two events that are detected within a pre-determined coincidence time window may be determined to be coincidence events (including true coincidence events as well as scatter and random coincidence events).

In step 1102, data associated with the coincidence events may be stored in a chronological list. That is, as the coincidence events are detected and identified, their data may be sequentially stored in a list according to their time of detection. The coincidence event data may include, for example, coordinates for the LOR (e.g., radial distance, angles), event time-stamps, and incident photon energy, or the like, or a combination thereof. In some embodiments, the coincidence event data may be histogrammed. In some embodiments, the coincidence event data may be arranged chronologically as they become available.

In step 1103, the list of coincidence event data may be optionally sorted based on one or more criteria. For example, the data may be sorted based on incident photon energy. Coincidence events that record certain photon energy may be grouped together to form a subset to facilitate an ordered subsets expectation maximization (OSEM) algorithm as described elsewhere in this disclosure. The data may also be sorted based on, for example, LOR angles or direct slice planes, or the like, or a combination thereof.

Iterative processing techniques such as Ordered Sub-set Expectation Maximization (OSEM) have been developed as a way of accelerating iterative reconstruction algorithms. Ordered Subset (OS) methods are based on performing at least the first few iterations (and optionally most or all of the iterations) on a smaller subset of the total available dataset. It may be necessary for the conversion of the iterative process that the symmetry of the subset be similar to the symmetry of the dataset as a whole.

Merely by way of example, the data may be arranged as a set of angular 2D projections. Using the OS algorithm, the projections within the dataset may be divided into five subsets. A first subset contains projections 1, 6, 11, ... , and 56 taken at 3, 18, 33, ... degrees. A second sub-set contains projections 2, 7, 12, ... , and 57. Continuing the pattern, a fifth subset contains projections 5, 10, 15, ... , and 60. As each iteration is performed using one sub-set which is a portion of the total dataset, the computation time is shorter.

Imaging systems having multiple smaller-sized detectors are desirable as patient data may be acquired more quickly. The multiple detectors may be arranged around a patient and may acquire data of the anatomy of interest simultaneously.

Coming back to step 1104, PET images may be computed by applying an adapted algorithm to the list of coincidence event data. Iterative algorithms may be used for PET image reconstruction. An iterative reconstruction algorithm may involve forward and/or backward projection of the coincidence event data.

It should be noted that the coincidence event data may be fed to the iterative update equations as soon as the data becomes available. Since the coincidence event data are being collected and stored chronologically, it may not be necessary to wait for the data acquisition to finish before starting the image reconstruction process. Instead, the reconstruction iteration may start after the data acquisition starts, making it possible to produce reconstructed PET images soon after the scanning is done. To incorporate corrections for scatter coincidence events, scatter sinograms may have to be generated first. However, generation of the scatter sinograms may only cause a short delay before the full list-mode image reconstruction may start.

The technique for reconstructing PET scan images in accordance with some embodiments of the present invention may be implemented in a computer-based system. The computer-based system may comprise one or more processors and/or computers capable of data manipulation, logic operation and mathematical calculation. The system may further comprise one or more storage devices for storing and managing PET scan raw data and coincidence event data, for example. In addition, a number of user interfaces may be provided for a user to initiate a reconstruction process and to view reconstructed PET scan images. The technique may be implemented on computers or computer networks.

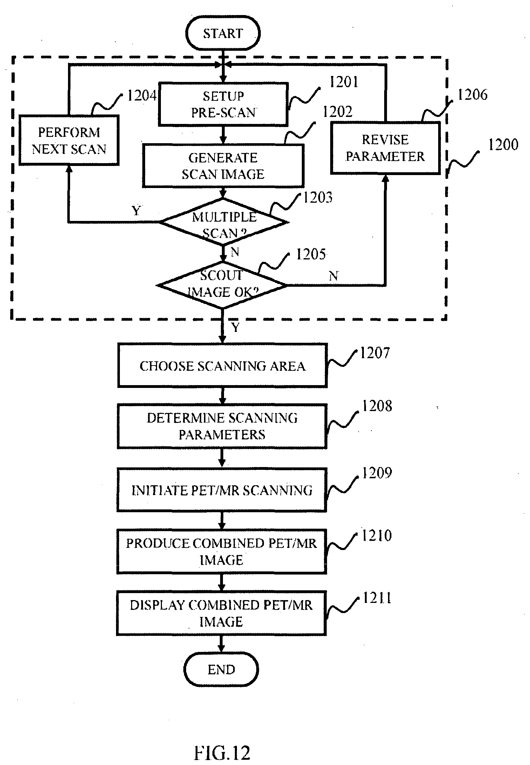

Referring to FIG. 12, in some embodiments, the multiple PET scout image generation is acquired as part of the PET scout image acquisition performed during the PET scan and indicated generally by dotted line 1200. Multiple PET scan image generation is an iterative process in which a set of scout images are acquired using a prescribed pulse sequence as indicated in step 1201 and then a navigator signal is acquired in step 1202. Views are acquired until all the views for one image are acquired in steps 1201, 1202, and 1204 as determined in step 1203. In some embodiments, step 1201 may be skipped.

If multiple PET scout images are determined to be unacceptable in step 1205, the relevant parameters for scanning a PET image as scout images need to be modified as indicated in step 1206. The various scout images for the patient at different bed positions are acquired as before and the process repeats until all the needed PET scout images are acquired.

In step 1207, as long as the subject including but not limited to the doctor or the patient, examines the PET scout images, a conventional planning of examination, such as PET and MR, may be decided to be performed on the patient, targeting on a designated area of the patient indicative of the area located in the scout image by the subject. Information from one or more of the scout images may be used to determine the scanning parameters used in the MR examination or PET examination in step 1208.

The scout image identifies the tissue position (plus, e.g., air) of each voxel in the field of view of the MR system and the PET scanner. In step 1209, a conventional MR examination or PET examination may be performed on the targeted area of the patient. In step 1210, a combined PET/MR image of the patient may be produced. This combined PET/MR image may then be output for displaying in step 1211.

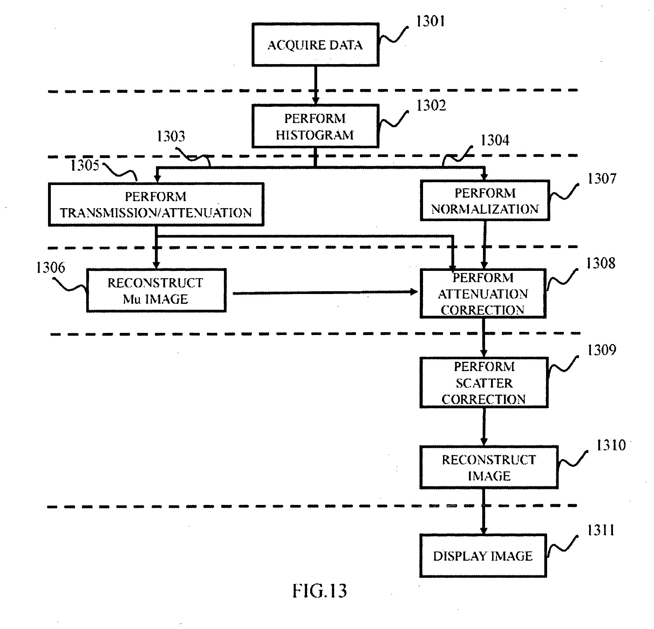

FIG. 13 is a block diagram that illustrates the parallel/pipelined architecture for PET image reconstruction. Although the following discussion is in terms of a process, at least some embodiments of the present invention includes the hardware and software used to implement the various process steps. The means to implement the individual processes are known in the art, as are the means to control the data flow between the processes. In one embodiment, the enumerated processes may be implemented by a multithreaded software program running on at least one computer. In another embodiment, a combination of hardware and software is used to implement the enumerated processes.

In FIG.13, the first block represents the acquisition 1301 of the data from the scanners. The acquisition process 1301 includes collecting the raw data from the scanner detectors and storing this data in a file of pre-determined data format. The data format may be for example list mode. The data acquired includes emission and/or transmission events along with information on the current position and/or height of the patient bed. The acquisition process 1301 may collect data continuously as the patient, on the patient bed, moves through the scanner. In other embodiments, the acquisition process 1301 may collect data discontinuously as the patient lying on the patient bed, moves through the scanner. The data from the acquisition process 1301 is output, simultaneously or asynchronously, to the histogram process 1302.

The histogram process 1302 creates a 3D sinogram space histogram of the emission and/or transmission events received from the acquisition process 1301, along with information on the current position of the patient bed. Those skilled in the art will recognize that the bed position information may be either a time signal based on a fixed bed speed or a position signal based on a bed position sensor. The emission events are histogrammed into a 3D sinogram space based on the current patient bed position. In some embodiments, when the patient bed has moved a predetermined amount, the histogramming is shifted a corresponding amount. In other embodiments, the patient bed may move an amount according to the specified histogramming data amount. With this shift, a portion of the 3D sinogram space is no longer within the histogramming region, which corresponds to the portion of the patient and patient bed that has traversed, and is no longer within, the axial field of view of the tomograph.

The histogram process 1302 outputs synchronous and/or asynchronous data as two data streams 1303, 1304. The first data stream 1303 from the histogram process 1302 transfers the contents of a transmission data file created during the histogram process 1302 to a transmission/attenuation process 1305. The transmission data file contains two-dimension (2D) data. The transmission/attenuation process 1305 uses an existing blank transmission data file to create an attenuation data file. The transmission/attenuation process 1305 outputs a data stream to both an attenuation correction process 1308 and a Mu image reconstruction process 1306. The Mu image reconstruction process 1306 creates a Mu image data file and outputs a data stream to an attenuation correction process 1308.

The second data stream 1304 transfers the contents of a 3D emission data file created during the histogram process 1302. The second data stream 1304 transfers the data to a normalization process 1307. The normalization process 1307 uses an existing normalization file to create a second emission data file. The existing normalization file contains the normalization coefficients. The normalization process 1307 outputs a data stream to the attenuation correction process 1308.

The attenuation correction process 1308 accepts a data stream from the transmission/attenuation process 1305 and Mu image reconstruction process 1306 and the normalization process 1307. The attenuation correction process 1308 creates a sinogram data file and outputs a data stream to a scatter correction process 1309, which creates an image data file and outputs a 3D data stream to an image reconstruction process 1310.

In some embodiment, the image reconstruction process 1310 may employ an iterative type process, for example a 3D OSEM process, or an analytic reconstruction process, for example a 3DRP process.

The data passing through the scatter correction process 1309 corresponds to the bed movement. After the patient bed has moved a predetermined amount, a portion of the 3D sinogram space is no longer within the scatter correction processing 1309 region. This portion of the 3D sinogram space corresponds to the portion of the patient and patient bed that has traversed, and is no longer within, the axial field-of-view of the tomograph. The output of the scatter correction process 1309 is transferred to an image reconstruction process 1310. After the reconstruction process 1310 is completed, the image is stored, and/or displayed at image display 1311.

All stages of the above-described parallel/pipelined architecture may be operating on data at the same time. In some embodiments, the data for a given processing stage may be different from the data in the other processing stages. Each stage of processing may complete processing the current data before accepting new data. Therefore, the data from one stage of processing may not be sent to the next stage of processing until the next stage has completed processing data from the previous cycle. Those skilled in the art will recognize that processing stages may be omitted or additional processing stages (various corrections, such as arc correction, etc.) may be added to the architecture without departing from the scope of the present invention.

Further, elements and/ or features of different example embodiments may be combined with each other and/or substituted for each other within the scope of this disclosure.

Still further, any one of the above-described and other example features of the present invention may be embodied in the form of an apparatus, method, system, computer program, computer readable medium and computer program product. For example, of the aforementioned methods may be embodied in the form of a system or device, including, but not limited to, any of the structure for performing the methodology illustrated in the drawings.

Even further, any of the aforementioned methods may be embodied in the form of a program. The program may be stored on a computer readable medium and is adapted to perform any one of the aforementioned methods when run on a computer device (a device including a processor). Thus, the storage medium or computer readable medium, is adapted to store information and is adapted to interact with a data processing facility or computer device to execute the program of any of the above mentioned embodiments and/ or to perform the method of any of the above mentioned embodiments. The computer readable medium or storage medium may be a built-in medium installed inside a computer device main body or a removable medium arranged so that it may be separated from the computer device main body. Examples of the built-in medium include, but are not limited to, rewriteable non-volatile memories, such as ROMs and flash memories, and hard disks. Examples of the removable medium include, but are not limited to, optical storage media such as CD-ROMs and DVDs; magneto-optical storage media, such as MOs; magnetism storage media, including but not limited to floppy disks (trademark), cassette tapes, and removable hard disks; media with a built-in rewriteable non-volatile memory, including but not limited to memory cards; and media with a built-in ROM, including but not limited to ROM cassettes; etc. Furthermore, various information regarding stored images, for example, property information, may be stored in any other form, or it may be provided in other ways.

**Claims**

1. An imaging method comprising:

   defining image regions on an object (102), each region being at a different bed position;
   setting imaging conditions for the defined image regions;
   acquiring, by data acquisition, a PET scout image for each of the defined image regions according to the set imaging conditions;

   wherein the plural PET scout images are combined together to form a scout image, used to pre-scan the object (102) for further medical examination,
   wherein the acquired PET scout image for each of the defined image regions is an iteratively reconstructed PET image, and
   wherein the data acquisition of the plural PET scout images does not finish before starting the scout image reconstruction and the reconstruction iteration starts after the data acquisition starts.

2. The method of claim 1, wherein the set imaging conditions include information about a plurality of protocols for each of the defined image regions.

3. The method of claim 1 or claim 2, wherein the set imaging conditions comprise an indicator for determining the resolution and sensitivity of the scout PET image.

4. The method of any one of claims 1-3, wherein the set imaging conditions include a movement speed of a bed on which the object (102) is located, and the movement speed of the bed is set differently for each of the defined image regions.

5. The method of any one of claims 1-4, wherein the time window for scanning each of the defined image regions is within fifteen seconds.

6. The method of any one of claims 1-5, wherein the defining the image regions comprises: automatically setting at least one of an imaging start point and an imaging end point for each of the defined image regions by using an attachment affixed to the object (102) or to the bed on which the object (102) is placed.

7. The method of any one of claims 1-5, wherein the defining the image regions comprises: setting at least one of an imaging start position and an imaging end position for each of the defined image regions by using a signal input through an external input device.

8. The method of claim 7, further comprising:
   setting the imaging start point by the external input device; and the imaging end point when initiating and terminating the signal through the external input device, respectively, wherein the signal is supplied for a time period.

9. The method of claim 7 or claim 8, wherein the external input device is built in the bed on which the object (102) is located or in an operating console.

10. The method of any one of claims 7-9, wherein the external input device comprises at least one of a button, a joystick, a touch panel, a switch, and a sensor.

11. An imaging apparatus (100) comprising:

an image region definer configured to define image regions on an object (102), each region being at a different bed position;
an imaging condition setter configured to set imaging conditions for the defined image regions;
an image processor configured to acquire a PET scout image for each of the defined image regions by data acquisition according to the set imaging conditions,

wherein the plural PET scout images are combined together to form a scout image, used to pre-scan the object for further medical examination;
wherein the acquired PET scout image for each of the defined image regions is an iteratively reconstructed PET image; and
wherein the data acquisition of the plural PET images does not finish before starting the image reconstruction process and the reconstruction iteration starts after the data acquisition starts.

12. The apparatus (100) of claim 11, wherein the set imaging conditions include a movement speed of a bed on which the object (102) is located, and the movement speed of the bed is set differently for each of the defined image regions.

13. The apparatus (100) of claim 11 or claim 12, wherein the image region definer automatically sets at least one of an imaging start point and an imaging end point for each of the defined image regions by using an attachment affixed to the object (102) or to the bed on which the object (102) is placed.

14. The apparatus (100) of claim 11 or claim 12, further comprising an external input device, wherein the image region definer is configured to set at least one of an imaging start position and an imaging end position for each of the defined image regions by using a signal which is input via the external input device.

15. The apparatus (100) of claim 14, wherein the image region definer is configured to set the imaging start point and the imaging end point when initiating and terminating the signal through the external input device, respectively, and the signal is supplied for a time period.

**Patentansprüche**

1. Bildgebungsverfahren, das Folgendes umfasst:

Definieren von Bildregionen eines Objekts (102), wobei sich jede Region in einer anderen Bettposition befindet;
Einstellen von Bildgebungsbedingungen für die definierten Bildregionen;
Erfassen eines PET-Scout-Bildes für jede der definierten Bildregionen mittels Datenerfassung gemäß den eingestellten Bildgebungsbedingungen;
wobei die mehreren PET-Scout-Bilder miteinander kombiniert werden, um ein Scout-Bild zu bilden, das zum Vorscannen des Objekts (102) zur weiteren medizinischen Untersuchung verwendet wird,
wobei das erfasste PET-Scout-Bild für jede der definierten Bildregionen ein wiederholt rekonstruiertes PET-Bild ist und
wobei die Datenerfassung der mehreren PET-Scout-Bilder erst beendet ist, wenn die Scout-Bildrekonstruktion gestartet wird, und die Rekonstruktionswiederholung gestartet wird, nachdem die Datenerfassung gestartet wurde.

2. Verfahren nach Anspruch 1, wobei die eingestellten Bildgebungsbedingungen Informationen über eine Vielzahl von Protokollen für jede der definierten Bildregionen beinhalten.

3. Verfahren nach Anspruch 1 oder Anspruch 2, wobei die eingestellten Bildgebungsbedingungen einen Indikator zum Bestimmen der Auflösung und der Empfindlichkeit des Scout-PET-Bildes umfassen.

4. Verfahren nach einem der Ansprüche 1-3, wobei die eingestellten Bildgebungsbedingungen eine Bewegungsgeschwindigkeit eines Bettes, auf dem sich das Objekt (102) befindet, beinhalten und die Bewegungsgeschwindigkeit des Bettes für jede der definierten Bildregionen unterschiedlich eingestellt ist.

5. Verfahren nach einem der Ansprüche 1-4, wobei das Zeitfenster zum Scannen von jeder der definierten Bildregionen innerhalb von fünfzehn Sekunden liegt.

6. Verfahren nach einem der Ansprüche 1-5, wobei das Definieren der Bildregionen Folgendes umfasst: automatisches Einstellen von mindestens einem eines Bildgebungsstartpunkts und eines Bildgebungsendpunkts für jede der definierten Bildregionen unter Verwendung eines Apparats, der am Objekt (102) oder am Bett, auf dem das Objekt (102) platziert ist, befestigt wird.

7. Verfahren nach einem der Ansprüche 1-5, wobei das Definieren der Bildregionen Folgendes umfasst: Einstellen von mindestens einem einer Bildgebungsstartposition und einer Bildgebungsendposition für jede der definierten Bildregionen unter Verwendung eines Signals, das über eine externe Eingabevorrichtung eingegeben wird.

8. Verfahren nach Anspruch 7, das ferner Folgendes umfasst: Einstellen des Bildgebungsstartpunkts über die externe Eingabevorrichtung und des Bildgebungsendpunkts, wenn das Signal über die externe Eingabevorrichtung initiiert bzw. beendet wird, wobei das Signal für eine Zeitperiode zugeführt wird.

9. Verfahren nach Anspruch 7 oder Anspruch 8, wobei die externe Eingabevorrichtung in das Bett, auf dem sich das Objekt (102) befindet, oder in eine Bedienkonsole eingebaut ist.

10. Verfahren nach einem der Ansprüche 7-9, wobei die externe Eingabevorrichtung mindestens eines von einer Taste, einem Joystick, einem Touchpanel, einem Schalter und einem Sensor umfasst.

11. Bildgebungseinrichtung (100), die Folgendes umfasst:

einen Bildregionsdefinierer, der dazu ausgelegt ist, Bildregionen eines Objekts (102) zu definieren, wobei sich jede Region in einer anderen Bettposition befindet; einen Bildgebungsbedingungseinsteller, der dazu ausgelegt ist, Bildgebungsbedingungen für die definierten Bildregionen einzustellen, einen Bildprozessor, der dazu ausgelegt ist, mittels Datenerfassung für jede der definierten Bildregionen ein PET-Scout-Bild gemäß den eingestellten Bildgebungsbedingungen zu erfassen, wobei die mehreren PET-Scout-Bilder miteinander kombiniert werden, um ein Scout-Bild zu bilden, das zum Vorscannen des Objekts zur weiteren medizinischen Untersuchung verwendet wird; wobei das erfasste PET-Scout-Bild für jede der definierten Bildregionen ein wiederholt rekonstruiertes PET-Bild ist und wobei die Datenerfassung der mehreren PET-Bilder erst beendet ist, wenn der Bildrekonstruktionsprozess gestartet wird, und die Rekonstruktionswiederholung gestartet wird, nachdem die Datenerfassung gestartet wurde.

12. Einrichtung (100) nach Anspruch 11, wobei die eingestellten Bildgebungsbedingungen eine Bewegungsgeschwindigkeit eines Bettes, auf dem sich das Objekt (102) befindet, beinhalten und die Bewegungsgeschwindigkeit des Bettes für jede der definierten Bildregionen unterschiedlich eingestellt ist.

13. Einrichtung (100) nach Anspruch 11 oder Anspruch 12, wobei der Bildregionsdefinierer unter Verwendung eines Apparats, der am Objekt (102) oder am Bett, auf dem das Objekt (102) platziert ist, befestigt wird, für jede der definierten Bildregionen automatisch mindestens eines von einem Bildgebungsstartpunkt und einem Bildgebungsendpunkt einstellt.

14. Einrichtung (100) nach Anspruch 11 oder Anspruch 12, die ferner eine externe Eingabevorrichtung umfasst, wobei der Bildregionsdefinierer dazu ausgelegt ist, unter Verwendung eines Signals, das via die externe Eingabevorrichtung eingegeben wird, für jede der definierten Bildregionen mindestens eines von einer Bildgebungsstartposition und einer Bildgebungsendposition einzustellen.

15. Einrichtung (100) nach Anspruch 14, wobei der Bildregionsdefinierer dazu ausgelegt ist, den Bildgebungsstartpunkt und den Bildgebungsendpunkt einzustellen, wenn das Signal über die externe Eingabevorrichtung initiiert bzw. beendet wird, und das Signal für eine Zeitperiode zugeführt wird.

**Revendications**

1. Procédé d'imagerie comprenant :

   la définition de régions d'images sur un objet (102), chaque région étant au niveau d'une position de plateau différente ;
   le réglage des conditions d'imagerie pour les régions d'images définies ;
   l'acquisition, par une acquisition de données, d'une image de repérage PET pour chacune des régions d'images définies en fonction des conditions d'imagerie réglées ; dans lequel les plusieurs images de repérage PET sont combinées ensemble pour former une image de repérage, utilisée pour pré-scanner l'objet (102) pour un examen médical supplémentaire,
   dans lequel l'image de repérage PET acquise pour chacune des régions d'images définies est une image PET reconstruite de manière itérative, et
   dans lequel l'acquisition de données des plusieurs images de repérage PET ne finit pas avant le début de la reconstruction d'images de repérage et l'itération de reconstruction commence après le début de l'acquisition de données.

2. Procédé selon la revendication 1, dans lequel les conditions d'imagerie réglées incluent des informations concernant une pluralité de protocoles pour chacune des régions d'images définies.

3. Procédé selon la revendication 1 ou la revendication 2, dans lequel les conditions d'imagerie réglées comprennent un indicateur pour déterminer la résolution et la sensibilité de l'image PET de repérage.

4. Procédé selon l'une quelconque des revendications 1 à 3, dans lequel les conditions d'imagerie réglées incluent une vitesse de mouvement d'un plateau sur lequel l'objet (102) est situé, et la vitesse de mouvement du plateau est réglée différemment pour chacune des régions d'images définies.

5. Procédé selon l'une quelconque des revendications 1 à 4, dans lequel la fenêtre temporelle permettant de scanner chacune des régions d'images définies est comprise dans les quinze secondes.

6. Procédé selon l'une quelconque des revendications 1 à 5, dans lequel la définition des régions d'images comprend :
   le réglage de manière automatique d'au moins un d'un point de début d'imagerie et d'un point de fin d'imagerie pour chacune des régions d'images définies en utilisant une fixation fixée à l'objet (102) ou au plateau sur lequel l'objet (102) est placé.

7. Procédé selon l'une quelconque des revendications 1 à 5, dans lequel la définition des régions d'images comprend :
   le réglage d'au moins une d'une position de début d'imagerie et d'une position de fin d'imagerie pour chacune des régions d'images définies en utilisant une entrée de signal à travers un dispositif d'entrée externe.

8. Procédé selon la revendication 7, comprenant en outre :
   le réglage du point de début d'imagerie par le dispositif d'entrée externe ; et du point de fin d'imagerie lors de l'initiation et de la fin du signal à travers le dispositif d'entrée externe, respectivement, le signal étant fourni pour une période de temps.

9. Procédé selon la revendication 7 ou la revendication 8, dans lequel le dispositif d'entrée externe est construit dans le plateau sur lequel l'objet (102) est situé ou dans une console d'exploitation.

10. Procédé selon l'une quelconque des revendications 7 à 9, dans lequel le dispositif d'entrée externe comprend au moins un élément parmi un bouton, une manette de commande, un panneau tactile, un commutateur et un détecteur.

11. Appareil d'imagerie (100) comprenant :

   un dispositif de définition de régions d'images configuré pour définir des régions d'images sur un objet (102), chaque région étant au niveau d'une position de plateau différente ;
   un dispositif de réglage de conditions d'imagerie configuré pour régler des conditions d'imagerie pour les régions d'images définies ;
   un processeur d'image configuré pour acquérir une image de repérage PET pour chacune des régions d'images définies par une acquisition de données en fonction des conditions d'imagerie réglées,

dans lequel la pluralité d'images de repérage PET sont combinées ensemble pour former une image de repérage, utilisée pour pré-scanner l'objet pour un examen médical supplémentaire ;

dans lequel l'image de repérage PET acquise pour chacune des régions d'images définies est une image PET reconstruite de manière itérative ; et

dans lequel l'acquisition de données des plusieurs images PET ne finit pas avant le début du processus de reconstruction d'images et l'itération de reconstruction commence après le début de l'acquisition de données.

12. Appareil (100) selon la revendication 11, dans lequel les conditions d'imagerie réglées incluent une vitesse de mouvement d'un plateau sur lequel l'objet (102) est situé, et la vitesse de mouvement du plateau est réglée différemment pour chacune des régions d'images définies.

13. Appareil (100) selon la revendication 11 ou la revendication 12, dans lequel le dispositif de définition de régions d'images règle automatiquement au moins un d'un point de début d'imagerie et d'un point de fin d'imagerie pour chacune des régions d'images définies en utilisant une fixation fixée à l'objet (102) ou au plateau sur lequel l'objet (102) est placé.

14. Appareil (100) selon la revendication 11 ou la revendication 12, comprenant en outre un dispositif d'entrée externe, dans lequel le dispositif de définition de régions d'images est configuré pour régler au moins une d'une position de début d'imagerie et d'une position de fin d'imagerie pour chacune des régions d'images définies en utilisant un signal qui est entré par l'intermédiaire du dispositif d'entrée externe.

15. Appareil (100) selon la revendication 14, dans lequel le dispositif de définition de régions d'images est configuré pour régler le point de début d'imagerie et le point de fin d'imagerie lors de l'initiation et de la fin du signal à travers le dispositif d'entrée externe, respectivement, le signal étant fourni pour une période de temps.

FIG.1

FIG.2-A

FIG.2-B

EP 3 224 801 B1

FIG.3-B

FIG.3-A

FIG.4

FIG.5

FIG. 6-A

FIG. 6-B

FIG. 6-C

FIG. 6-D

START

DETECT RESPONSE RAYS — 701

EXTRACT SINOGRAMS — 702

STORE SINOGRAMS — 703

RECONSTRUCT HIGH-RESOLUTION IMAGE — 704

END

FIG.7

801 — RECORD PET SCOUT IMAGE

802 — PERFORM PET PLANNING

803 — PERFORM MR PLANNING

804 — RECORD PET MEASUREMENT DATA

805 — RECORD MR IMAGE

806 — ADDITIONAL PET RECORDING

807 — PERFORM IMAGING

FIG.8

901 — SIGNAL CLASSIFIER

902 — FIRST IMAGE GENERATOR

CONTROL UNIT

903 — PARAMETER MEASUREMENT UNIT

904 — SECOND IMAGE GENERATOR

FIG.9

START

CLASSIFY INPUT SIGNALS BASED ON POSITION OF PET — 1001

GENERATE FIRST IMAGE SET BY RECONSTRUCTING INPUT SIGNALS — 1002

MEASURE PSF — 1003

GENERATE SECOND IMAGE INFORMATION BASED ON PSF AND FIRST IMAGE SET — 1004

END

FIG.10

START

DETECT COINCIDENCE EVENTS — 1101

STORE DATA ASSOCIATED WITH THE COINCIDENCE EVENTS IN A LIST — 1102

SORT THE LIST OF EVENTS POSSIBLY BASED ON ENERGY OR LOR ANGLES — 1103

COMPUTE PET IMAGES USING ADAPTED ALGORITHM TO THE LIST, INCORPORATING CORRECTION DATA FOR SCATTER AND RANDOM EVENTS — 1104

END

FIG.11

FIG.12

FIG.13

scanner surface

point source

x

y

2D ring scanner

z

x

3D cylinder scanner

FIG.14

**EP 3 224 801 B1**

**Patent documents cited in the description**

- US 20070173716
- US 2013105699 A1